# EUROPEAN PATENT APPLICATION

(11) **EP 1 498 143 A1**
(43) Date of publication of application: **19.01.2005**
(21) Application number: 03291798.1
(22) Date of filing: 18.07.2003
(51) Int. Cl.: A61K 47/44, A61K 9/107, A61K 47/14, A61K 31/337

(54) **Self-emulsifying and self-microemulsifying formulations for the oral administration of taxoids**

(71) Applicant: Aventis Pharma S.A., 92160 Antony (FR)
(72) Inventor: Peracchia, Maria-Teresa, 75005 Paris (FR); Cote, Sophie, 92160 Antony (FR); Gaudel, Gilbert, 75013 Paris (FR)
(74) Representative: Le Pennec, Magali

(57) **Abstract**

Self-emulsifying and self-microemulsifying formulations for the oral administration of taxoids. The present invention relates to novel formulations of taxoids for oral administration.

## Description

The present invention relates to oral formulations of taxoids.

The taxoids used in the formulations according to the invention are preferably of the general formula (I) 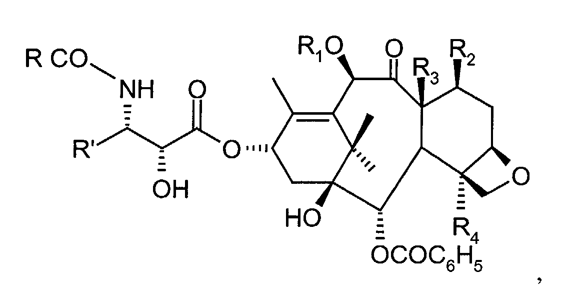 wherein
R₁ is H, acyl (C₂-C₄), alkyl (C₁-C₃),
R₂ is OH, alkoxy or R₂ and R₃ are methylene,
R₃ is CH₃ or R₂ and R₃ are methylene,
R₄ is OCOCH₃ or OCOOCH₃,
R is phenyl or alkoxy (C₃-C₄) or alkenyloxy (C₃-C₄), preferably phenyl or tert-butoxy,
R' is aryl, preferably phenyl, optionally substituted or alkyl (C₂-C₄) or alkylen (C₂-C₄).

The taxoids used in the formulations according to the invention are for example the taxoids of formula (Ia) to (If) below

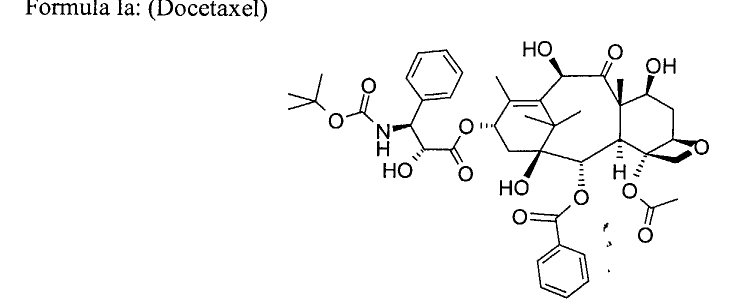

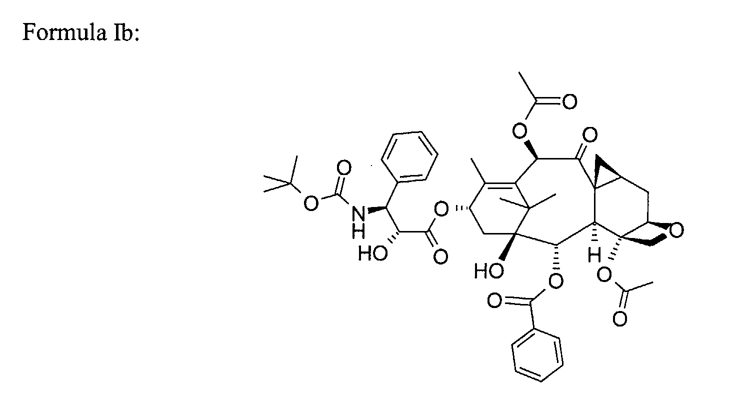

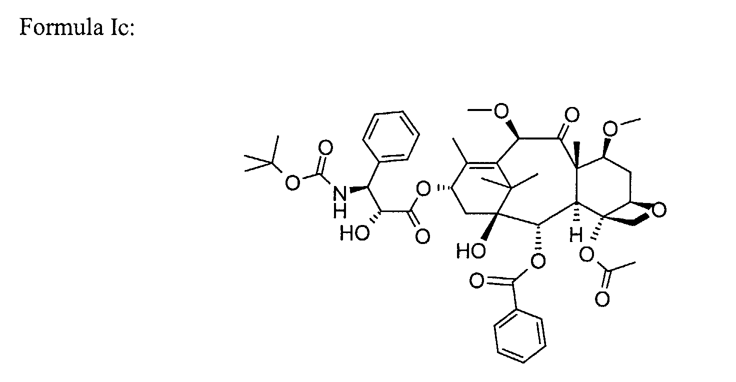

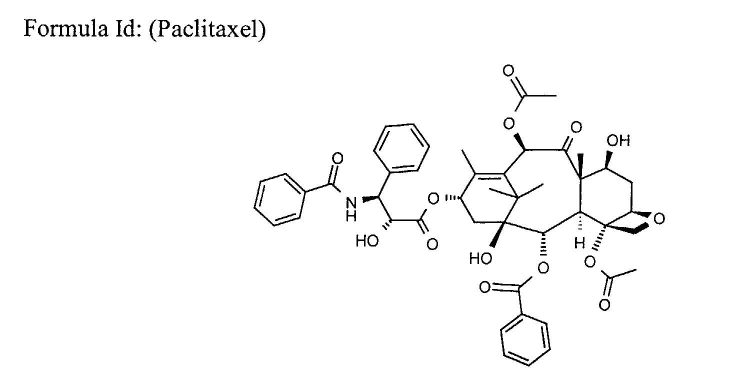

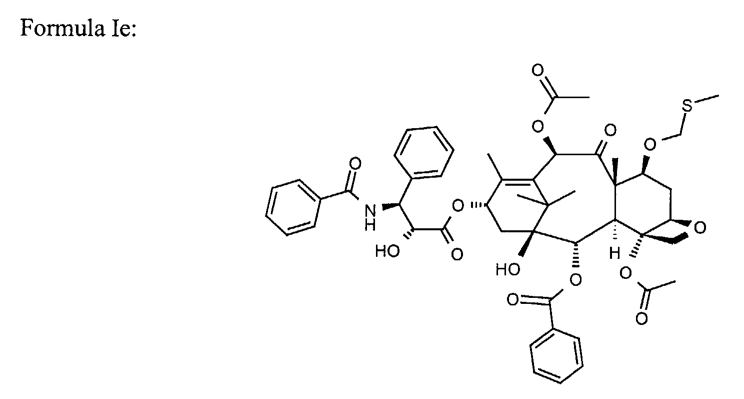

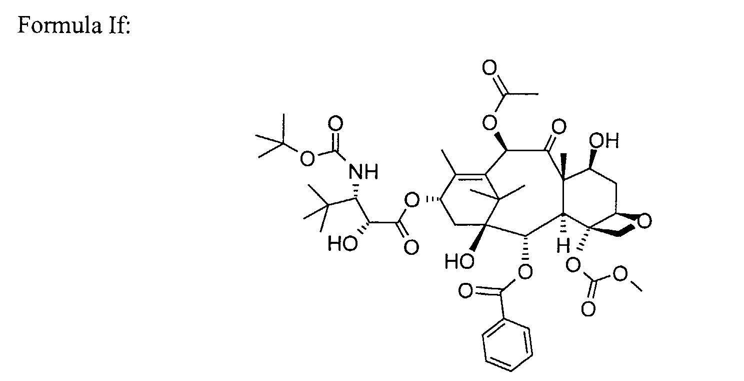

Taxoids of general formula (Ia) to (If) and their applications are known. These taxoids are particularly advantageous for their use as chemotherapeutic agents. Unfortunately, taxoids are poorly water-soluble compounds. The molecules are slightly lipophilic with a relatively high molecular weight. Up until now taxoids are administered intravenously, in particular using formulations consisting of PS80 or cremophor at high content. It was the aim of the current invention to develop taxoid formulations for oral administration.

Oral administration of PS80 or cremophor formulations of taxoids led to an extremely low bioavailability in animals probably because of a high metabolism rate, like e.g. dogs. In addition, formulations consisting of a high content of PS80 (e.g. less than 40 mg taxoid/g PS80) are not desirable for oral administration because of the potential toxicity of PS80 in contact with the intestinal mucosa. Furthermore, a dose escalation study would not be possible with the expected doses because of the solubility limit and as a consequence the limited PS80 solubilisation capacity for taxoids in gastro-intestinal fluids. Finally, the pharmaceutical development of a drug dosage form would be a main issue: indeed, the extemporary dilution of the PS80 solution with an aqueous medium is not envisageable for the oral administration of a cytotoxic agent.

Numerous documents describe systems suitable for solubilising and/or enhancing the bioavailability of hydrophobic active ingredients. However, the systems tested have so far proved ineffective for the preparation of pharmaceutical compositions containing taxoids which are stable and bioavailable and in which the taxoid can be administered orally at an effective concentration.

WO 95/24893 describes delivery systems for hydrophobic drugs. This application describes compositions comprising a digestible oil, a lipophilic surfactant and a hydrophilic surfactant that are intended for the formulation of hydrophobic active ingredients and for the enhancement of their bioavailability.

WO 99/49848 describes pharmaceutical dosage forms for anticancer drugs, e.g. paclitaxel in which the active drug is formulated as stable self-emulsifying preconcentrate. WO 99/49848 describes compositions comprising an anticancer drug in a carrier system comprising at least one hydrophobic component selected from tri-, di- or monoglycerides, free fatty acids, fatty acid esters or derivatives thereof, and a hydrophilic component selected from hydroxyalkane, dihydroxyalkane or polyethylene glycol (PEG), and comprising at least one surfactant.

EP 0 152 945 B1 describes transparent multi-component systems for pharmaceutical application containing one or several active ingredients in a system composed of an oil component, surfactants, co-surfactant and optionally water.

EP 0 670 715 B1 describes compositions for pharmaceutical use intended to be ingested, able to form a microemulsion, comprising at least an active ingredient, a lipophilic phase, a surfactant, a co-surfactant and a hydrophilic phase of special composition.

EP 0 334 777 B1 describes a micro-emulsion with pharmaceutical use comprising a water-soluble phase and a lipidic phase, comprising at least one surface-active agent based on Polyethylenglycol and at least one co-surfactant based on polyglycerol.

It has now been found, and that is what constitutes the subject of the present invention, that it is possible to prepare chemically and physically stable formulations of taxoid for oral administration. The present invention relates to a self-emulsifying formulation for the oral administration of taxoids comprising at least one taxoid and at least one amphiphilic surfactant with hydrophilic character that is preferably Labrasol® (glyceride of PEG and saturated fatty acids).

In a preferred embodiment of the invention the formulation contains a taxoid up to 200 mg/ml Labrasol®, for example 150 mg taxoid per ml Labrasol®, preferably between 5 and 100 mg taxoid per ml Labrasol®, e.g. 5 mg/ml, 10 mg/ml, 20 mg/ml, 30 mg/ml, 40 mg/ml, 50 mg/ml, 60 mg/ml, 70 mg/ml, 80 mg/ml, 90 mg/ml or 100 mg/ml.

The taxoid/ Labrasol® formulation may comprise further certain additional additives, the latter may be stabilizing agents, preservatives, agents which make it possible to adjust the viscosity, or agents that can modify, for example, the organoleptic properties.

In another aspect the invention relates to a self-microemulsifying (SMES) formulation for the oral administration of taxoids comprising at least one taxoid, Cremophor EL® (POE hydrogenated castor oil), at least one co-surfactant and at least one oil.

The co-surfactant is an amphiphilic surfactant with lipophilic character with an HLB (HLB stands for hydrophilic-lipophilic balance) of less than 10. The co-surfactant is preferably chosen from Peceol® (Glyceryl monooleate), Lauroglycol 129® (PG monolaurate), Capryol 90® (Polyethylenglycol monocaprylate), Maisine 35-1® (Glyceryl mono-dicaprylate) and Imwitor 988® (Glyceryl mono-dicapryl).

The oil is preferably a medium-chain triglyceride. The medium-chain triglyceride is preferably Miglyol 812N®.

The amount of co-surfactant is preferably less than 50 % (weight percent), more preferable less 40 %, for example 35%, 30%, 25%, 20%, 15%, 10% or 5%. The oil concentration is preferably less than 40 %, more preferably less than 30 %, for example 25%, 20%, 15%, 10% or 5%. In a preferred embodiment of the invention the ratio of surfactant to co-surfactant is 3:1 or higher (i.e. 5:1 or 6:1) and the oil concentration is 20%.

In a preferred embodiment of the invention the SMES formulation contains a taxoid in an amount comprised between 5 and 50 mg/g, preferably closer to 50 mg/g.

In a preferred embodiment of the invention the formulation has one of the following compositions:
- Cremophor EL/Maisine/Miglyol 812N or
- Cremophor EL/Lauroglycol 90/Miglyol 812N or
- Cremophor EL/Capryol 90/Miglyol 812N or
- Cremophor EL/Peceol/Miglyol 812N or
- Cremophor EL/Imwitor 988/Miglyol 812N.

In a preferred embodiment of the invention the formulation has one of the following compositions:
- Cremophor EL/Maisine/Miglyol 812N at 50 mg/g or
- Cremophor EL/Lauroglycol 90/Miglyol 812N at 50 mg/g or
- Cremophor EL/Capryol 90/Miglyol 812N at 50 mg/g or
- Cremophor EL/Peceol/Miglyol 812N at 50 mg/g or
- Cremophor EL/Imwitor 988/Miglyol 812N at 50 mg/g.

In a preferred embodiment of the invention the SMES contains 50 mg taxoid per g formulation, wherein the formulation comprises 60% Cremophor EL, 20% Imwitor 888 and 20% Miglycol 812N (weight percent).

The taxoid/SMES formulation may comprise further certain additional additives, the latter may be stabilizing agents, preservatives, agents which make it possible to adjust the viscosity, or agents that can modify, for example, the organoleptic properties.

In another aspect the invention relates to a process for preparing said self-emulsifying formulation, wherein there is prepared, where appropriate, the mixture of principal excipients, after heating, if necessary, in the case of the solid or semisolid excipients, and then, if necessary, the mixture with the additional additives, and then the taxoid and stirring is maintained in order to obtain a homogeneous mixture.

The strategy has been to obtain a formulation able to enhance taxoid solubilisation in aqueous medium by using amphiphilic- and lipid-based formulations able to form a colloidal system (fine emulsion or micellar solution) *in vivo.*
Among amphiphilic and lipid-based formulations, 3 categories were identified:
Amphiphilic polymers (micelle or emulsion formation)
Phospholipids (lipidic vesicles formation)
SMES (self-microemulsifying systems): oil + surfactant + co-surfactant (microemulsion formation)

After a first selection of proper excipients (in terms of safety and developpability), the solubility of taxoids in the excipient was the first screening step for the choice of the excipient and the selection of the prototypes. Then, the prototypes (liquid or semi-solid) were manufactured, and characterized in terms of *in vitro* behaviour in simulated GI media and chemical stability. Finally, the physical properties and stability of the semi-solid prototypes have been investigated.

Different categories of excipients described in the literature as components of amphiphilic and lipid-based formulations have been tested for the solubility of taxoids:
1. Oils (medium-chain triglycerides, fatty acids, ...)
2. Amphiphilic surfactants with hydrophilic character (HLB>10) (PEO sorbitan fatty acids, castor oil ethoxylates, fatty acid ethoxylates.)
3. Amphiphilic surfactants with lipophilic character (HLB<10) (glycerides of fatty acids: glyceryl oleate/linoleate, oleoyl macrogol glycerides; derivatives of propylene glycol: PG caprylate/linoleate,)
4. Phospholipids (lecithins)
5. Hydrophilic solvents (PEG 400,..)

All the selected excipients are described as safe for oral administration, and they are developable (alone or as mixture) as pharmaceutical dosage form (soft or hard capsule).

The chemical composition of the selected excipients in liquid form at room temperature, as well as the solubility of taxoid of formula Ib, are reported in table 1 below.

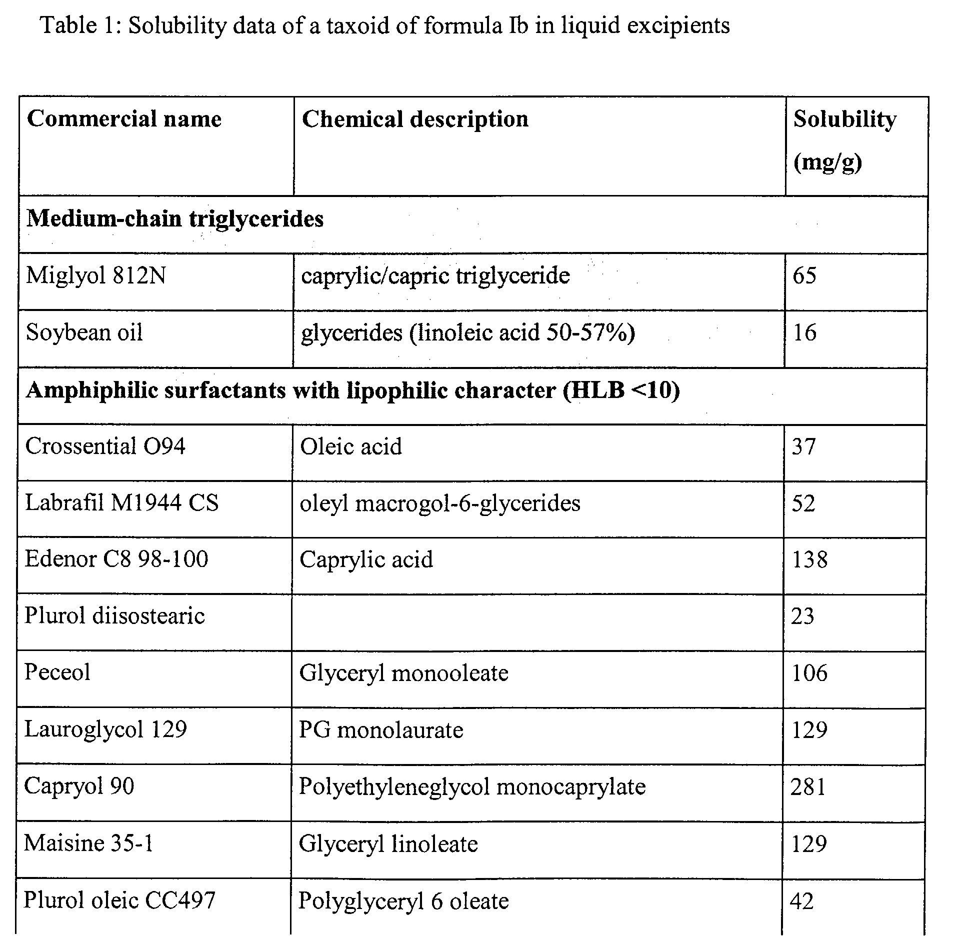

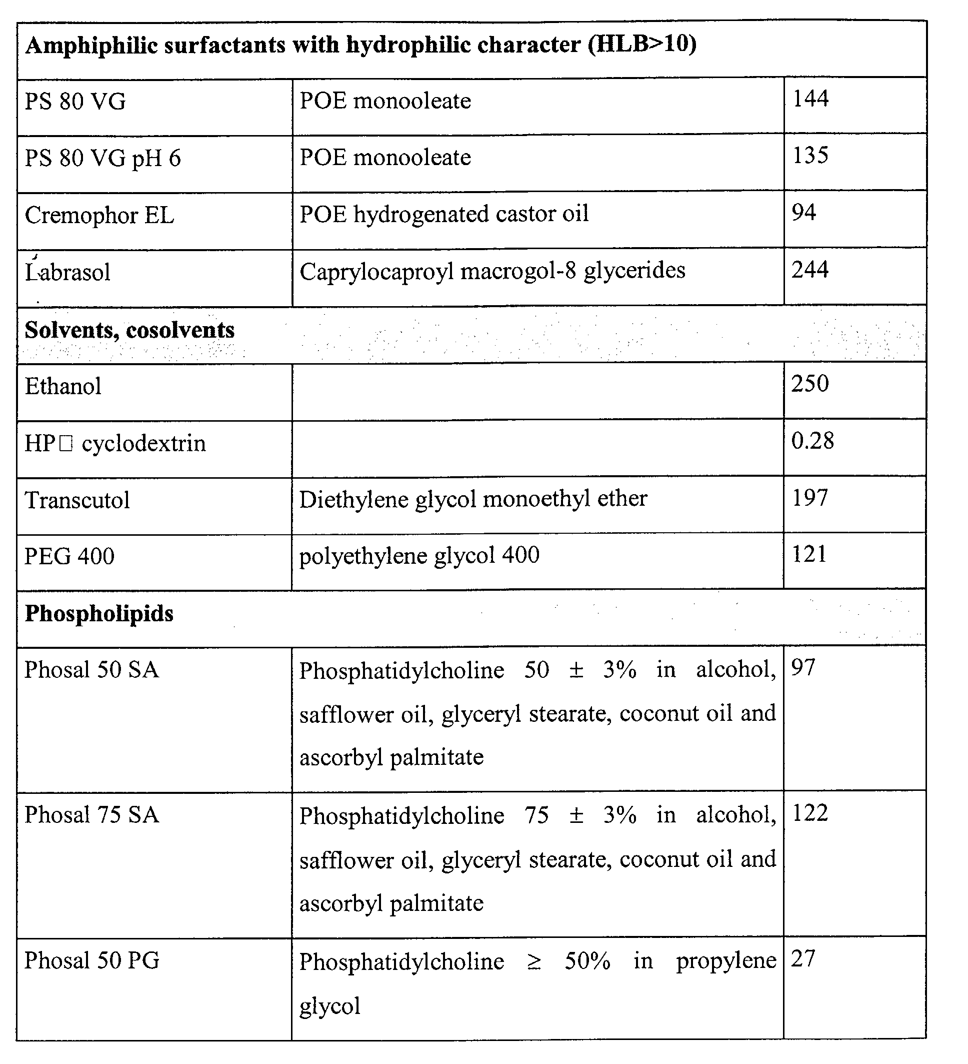

The following table 2 reports the chemical composition of the selected excipients in semi-solid form at room temperature, as well as the solubility of a taxoid of formula Ib. Excipients had been previously melted up to 70°C for drug dissolution.

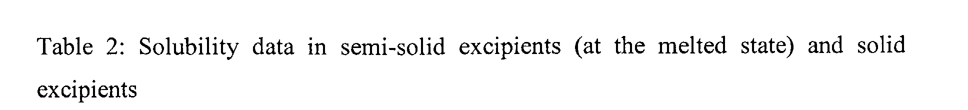

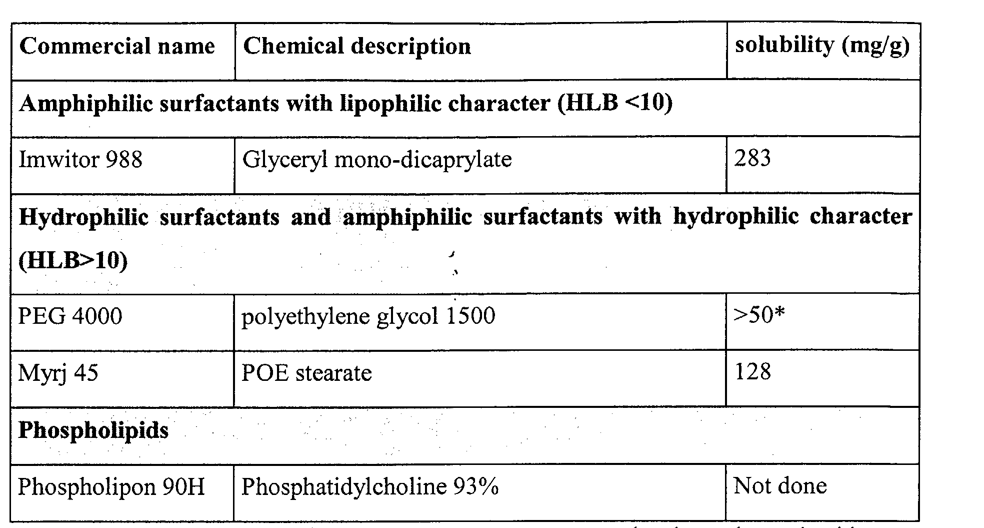

The solubility of taxoid of formula Ib at room temperature has been determined by X ray diffraction.

Taking into account the solubility of a taxoid of formula Ib, for the 3 categories of drug delivery systems the following excipients were retained:
Phosal 75SA and Phospholipon 90H for lipidic vesicle formation
Labrasol for emulsion formation
Microemulsion formation: as surfactant Myrj 45, PS80, Cremophor EL, Labrasol; as co-surfactant: Maisine, Capryol 90, Peceol, Lauroglycol 90, Imwitor 988; as oil: Miglyol 812N, Edenor.

For the first 2 categories, the excipients were formulated as binary systems with the drug, at the following concentrations:
- Phosal 75SA (solution): 100 mg/g formulation
- Phospholipon 90H (solid powder): 50, 100 mg/g formulation
- Labrasol (solution): 50, 100, 200 mg/g formulation

For the SMES category (3-components system), a first screening of the excipients as oil, surfactant (HLB>10) and co-surfactant (HLB<10), combined together at different ratios without the presence of the active, was necessary for identifying the formulations able to form a microemulsion (droplet size <30 nm) after infinite dilution with water. With this screening the following SMES were identified:
- Cremophor EL/Maisine/Miglyol 812N at 50 mg/g
- Cremophor EL/Lauroglycol 90/Miglyol 812N at 50 mg/g
- Cremophor EL/Capryol 90/Miglyol 812N at 50 mg/g
- Cremophor EL/Peceol/Miglyol 812N at 50 mg/g
- Cremophor EL/Imwitor 988/Miglyol 812N at 50 mg/g

The ratio between the excipients in the retained formulations was as follow: ratio surfactant to co-surfactant 3:1 and with oil concentration of 20%.

It is understood that the dosage may vary according to the degree or the nature of the condition to be treated. Thus, the quantity of active product in a composition according to the invention will be determined such that a suitable dosage can be prescribed. As a result, the quantity of taxoids varies as a function of its solubility in the mixture and also as a function of the appropriate dosage for the treatment of patients. Preferably, care should be taken not to load more than 10% w/w of taxoid drug so as to avoid microemulsion destabilization to occur.

It is understood that, to choose the most appropriate daily dosage in humans, there should be taken into account the weight of the patient, his general state of health, his age and all factors which may influence the efficacy of the treatment. Preferably, the compositions are prepared such that a unit dose contains from 0.1 to 50 mg of active product.

In the alternative, where a second active ingredient is introduced, the compositions may comprise 0.2 to 50 mg. However, this quantity may optionally be lower and may vary from 0.2 to 10 mg.

When the composition further comprises certain additional additives, the latter may be stabilizing agents, preservatives, agents which make it possible to adjust the viscosity, or agents that can modify, for example, the organoleptic properties.

The stabilizing agents may be, for example, antioxidants chosen in particular from α-tocopherol, ascorbyl palmitate, BHT (butyl hydroxytoluene), BHA (butyl hydroxyanisole), propyl gallate or malic acid for example.

The preservatives may, by way of example, be chosen from sodium metabisulfite, propylene glycol, ethanol or glycerin.

Among the agents capable of adjusting the viscosity, there may be mentioned, for example, lecithins, phospholipids, propylene glycol alginate, sodium alginate or glycerin.

The agents capable of modifying the organoleptic properties of the composition are, by way of example, malic acid, fumaric acid, glycerin, vanillin or menthol.
When such additives are used, the latter may constitute from 0.001% to 5% by weight of the total composition.

According to the invention, the pharmaceutical composition may be obtained by mixing, where appropriate, the principal excipients (after heating if necessary, in the case of solid or semisolid excipients), and then, if necessary, mixing with the additional additives, followed by the addition of the taxoid and maintaining stirred in order to obtain a homogeneous mixture.

The compositions according to the invention may be provided in the liquid, state.

They are particularly suitable for presentation in the form of hard gelatin capsules or soft gelatin capsules, or in the form of an oral solution.

The compositions according to the invention are particularly advantageous because of their good stability, both physically and chemically, and the enhancement of the bioavailablity which they offer upon oral administration of taxoids.

The following examples, given without limitation, illustrate formulations according to the present invention.

### Figures

Figure 1: Taxoid of formula Ib relase profile of different formulations at 100 mg/g in simulated gastric medium
Figure 2: Taxoid of formula Ib relase profile of self-microemulsifying system (SMES) at 50 mg/g in simulated gastric medium
Figure 3: Particle size of Taxoid of formula Ib formulations in simulated gastric medium
Figure 4: Particle size of Taxoid of formula Ib formulations leading to droplets <50 nm in simulated gastric medium
Figure 5: Taxoid of formula Ib - PK profiles with the PS80 formulation
Figure 6: Taxoid of formula Ib - PK profiles with the SMES formulation
Figure 7: Taxoid of formula Ib - PK profiles of nanocrystals of Taxoid of formula Ib
Figure 8: Taxoid of formula Ib - PK profiles of the 3 formulations in dog no 1
Figure 9: Taxoid of formula Ib - PK profiles of the 3 formulations in dog no 2
Figure 10: Taxoid of formula Ib - PK profiles of the 3 formulations in dog no 3
Figure 11: Taxoid of formula Ib - Comparison of plasma radioactivity Cmax of different formulations in Beagle dogs.
Figure 12: Taxoid of formula Ib - Comparison of plasma radioactivity AUC of different formulations in Beagle dogs.

### Examples

### Example 1: Preparation of Prototypes

### 1.1 Materials

- Taxoid of formula Ib
- Miglyol 812N (Condea Vista Company, Cranford, NJ, USA)
- Labrasol (Gattefossé, Saint Priest, F)
- Cremophor EL (BASF AG, Ludwigshafen, DE)
- Capryol 90 (Gattefossé, Saint Priest, F)
- Lauroglycol 90 (Gattefossé, Saint Priest, F)
- Peceol (Gattefossé, Saint Priest, F)
- Maisine 35-1 (Gattefossé, Saint Priest, F)
- Imwitor 988 (Condea Vista Company, Cranford, NJ, USA)
- Phosal 75SA (Nattermann, Cologne, DE)
- Phospholipon 90H (Nattermann, Cologne, DE)
- PS80 VG DF (Seppic, Paris, France)

### 1.2 Preparation of the solutions

The weighed drug was dispersed in the excipient, and then maintained under mechanical stirring until complete dissolution (approximately 3-5 hours). In the case of the SMES formulations, the drug was dissolved in the mix of the 3 excipients previously homogenized together.

### 1.3 Preparation of the solid dispersions.

The drug and the excipient (Phospholipon 90H) were dispersed in absolute ethanol (0.1 g drug, 0.9 g excipient, 6 g: ethanol) in a balloon reactor, than heated at 50°C until dissolution. The solvent evaporation by Rotavap (150-200 mbar, 1h30, 110 rpm rotation) led to the formation of a fluffy white powder.

### 1.4 Chemical stability

The chemical stability of the different formulations is a key parameter. Prototypes were stored in bulk (glass vial) for up to 3 months at +5°C (± 3°C), 25°C (± 2°C) and 30°C (± 2°C) under 60% (± 5%) relative humidity (RH) and 40°C (± 2°C) under 75% (± 5%) RH.
The stability was evaluated by mean of the potency determined by HPLC, as well as evaluation of relative substances. The prototypes analysed for drug dosage and stability studies are showed in the table below.

**Table 3:**

| Prototypes of taxoid of formula Ib formulations for stability study | |
|---|---|
| **PROTOTYPE** | **DRUG CONCENTRATION** **mg/g formulation** |
| PS 80 | 100 |
| Capryol 90 | 250 |
| Labrasol | 100 |
| Labrasol | 200 |
| Phosal 75 SA | 100 |
| CremophorEL-Miglyol 812N-Peceol | 50 |
| CremophorEL-Miglyol 812N -Maisine | 50 |
| CremophorEL-Miglyol 812N-Lauroglycol 90 | 50 |
| Cremophor EL-Miglyol 812N-Capryol 90 | 50 |
| Cremophor EL-Miglyol 812N-Imwitor 988 | 50 |
| Phospholipon 90 H | 50 |
| Phospholipon 90 H | 100 |

All the formulations are stable 3 months at 40°C under 75% RH, except the SMES formulations. Indeed, the SMES are stable 1 month at 25°C, whereas at 40°C the impurity taxoid of formula Ib (hydrolysis) appears (1.15-3.88% at t_{1 month}, depending on the nature of the co-surfactant). The 3 months analysis of the sample allowed to evaluate if this impurity increase was critical: after 3 months, an increase of taxoid of formula Ib impurity content was noticed. The SMES is stable at 5°C during 7 months.

### Example 2: in vitro behaviour in simulated gi media

Release profiles after incubation in simulated gi media

### Composition of the simulated fluids

The following simulated media were selected for the present experiment:
- Gastric medium USP, pH 1.2
- Fasted intestinal medium, pH 6.8 (ref. Dressman et al., Pharm. Res., 1998)
- Fed intestinal medium, pH 5 (ref. Dressman et al., Pharm. Res., 1998)

**Table 4:**

| Composition of the simulated gastro-intestinal media | | |
|---|---|---|
| **Gastric medium (G)** | | |
| Sodium chloride | 2 g | |
| Hydrogen chloride 1N | 100 ml approximately | |
| Demineralised water | qsp 1000 ml | |

| **Fasted intestinal medium (Fassif)** | | For 500 ml |
|---|---|---|
| Potassium hydrogenophosphate | 0.029 M | 1.97 g |
| Sodium hydroxide | qs pH 6.8 | qs pH 6.8 |
| Sodium Taurocholate | 5 mM | 1.34 g |
| Lecithin (Phospholipon 90G) | 1.5 mM | 0.58 g |
| Potassium chloride | 0.22 M | 8.2 g |
| Demineralised water | qsp 11 | qsp 500 ml |

| **Fed intestinal medium (Fessif)** | | For 500 ml |
|---|---|---|
| Acetic acid | 0.144 M | 4.33 g |
| Sodium hydroxide | qs pH 5 | qs pH 5 |
| Sodium Taurocholate | 15 mM | 4.03 g |
| Lecithin (Phospholipon 90G) | 4 mM | 1.55 g |
| Potassium chloride | 0.19 M | 7.08 g |
| Demineralised water | qsp 11 | qsp 500 ml |

### 2.1 Experimental conditions

In a first step of experiments, the formulations (100 mg drug/g formulation, 500 mg formulation in a hard gelatin capsule) were diluted 1:500 in the gastric medium (1 capsule/250 mL), than incubated 2 hours at 37°C under stirring (50 rpm) in a USP standard dissolution apparatus.
The same experiment has been carried out in gastric medium with 2 capsules loaded with less concentrated formulations (50 mg drug/g formulation), in order to study the effect of the drug/excipient and excipient/medium ratio on the release profile.
In a second step of experiments, a first incubation of 1 hour in gastric medium was followed by 2 hours incubation in fasted intestinal or fed intestinal medium, in order to simulate the gastric emptying process.

Samples were taken after 5-15-30-60 min and 2h. The drug concentration was determined by HPLC after centrifugation (6000 rpm, 10 min). Homogeneity of the medium was evaluated by sampling bottom, medium and top of the vessel.

### 2.2 Results

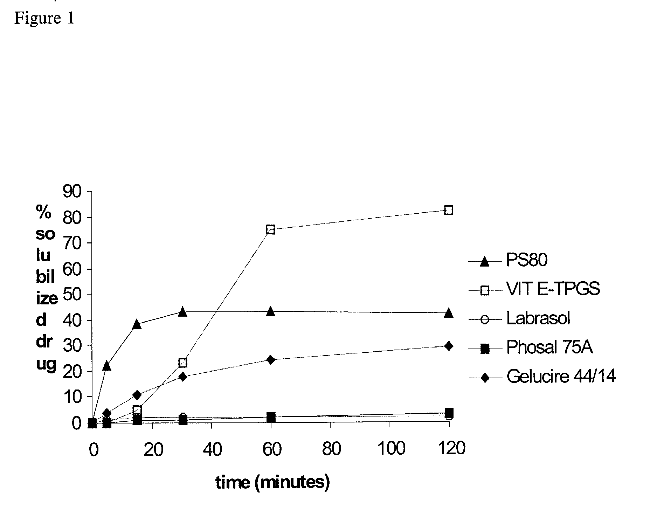

Drug release profiles in gastric medium of formulations at 100 mg/g are shown in the figure 1.
The profile obtained with formulation data from Phosal is not very representative, since these formulations led to the formation of a very heterogeneous mixture after incubation. The Labrasol formulation led to the formation of a very homogeneous emulsion with the medium, despite the low amount of drug recovered after centrifugation (see release profile), suggesting that for a coarse emulsion the centrifugation (determining the collapse of the emulsion) could sub-estimate its *in vitro* performance. The experiment with Phospholipon 90H was stopped (no data collection) since the powder floating did not allow the formation of a homogeneous suspension.

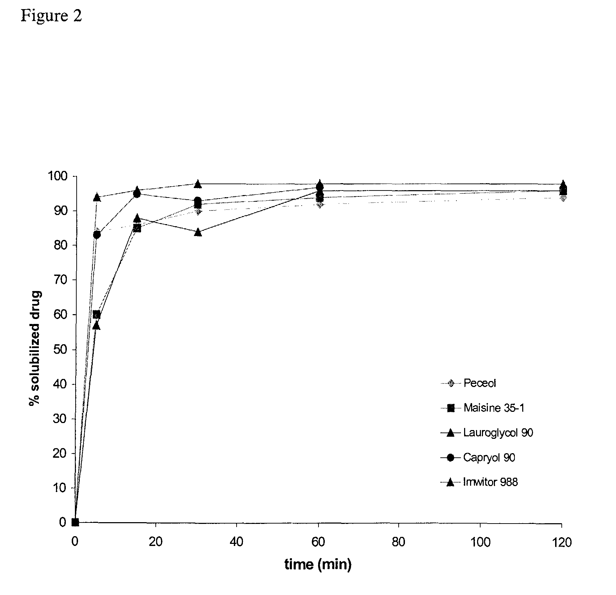

The *in vitro* profiles of all the 5 self-microemulsifying systems (SMES) tested exhibited a 100% "release" in a few minutes (figure 2). However, the fact that the centrifugation does not allow to separate the aqueous and oily phase of the SMES means that the SMES is still finely dispersed in the aqueous phase (gastric medium) after centrifugation, and the drug is still solubilised into the tiny microemulsion droplets. Nevertheless, the SMES system is definitely extremely interesting, even if the chemical stability could be an issue (effect of the presence of hygroscopic surfactant or co-surfactant on the drug chemical stability to investigate).

### Example 3: particle size analysis after incubation in gastric medium (USP)

The aim of this part of the study was to evaluate, by particle size measurement, the colloidal stability and the self-emulsifying properties of the emulsion/microemulsion/micellar solution of taxoid of formula Ib formulations after incubation in the gastric medium.

### 3.1 Experimental conditions

The formulations (concentration 100 mg drug/g formulation, 100 mg formulation) were diluted 1:500 in the gastric medium (50 mL), then incubated 2 hours at 37°C under mechanical stirring (300 rpm).
The sample was diluted immediately with water for size measurement or filtered onto 2 µm if necessary. The filtration allowed to retain oil droplets>2 µm, as well as drug crystals >2 µm, in order to allow the particle size measurement by QELS (quasi-elastic light scattering) (Nanosizer N4+, Beckmann-Coulter).

### 3.2 Results

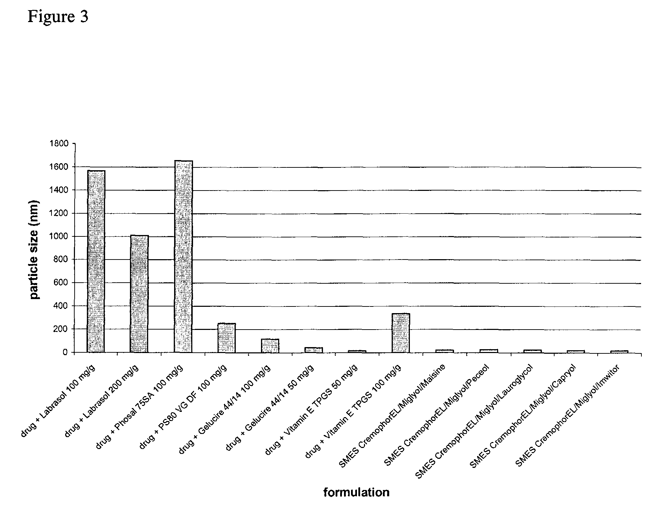

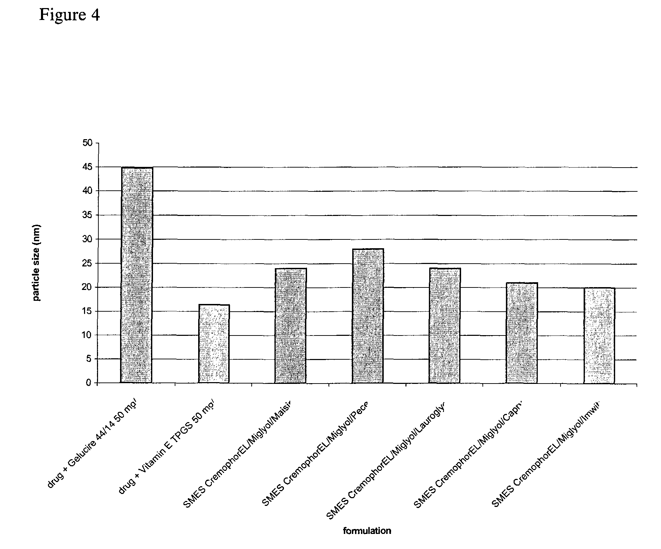

As shown in the figures 3 and 4, a particle size <50 nm was obtained only in the case of the formulations with active concentration of 50 mg/g: the 5 microemulsions (nevertheless their composition).
The results suggest using the formulations able to form small and monodisperse droplets in gastric medium in order to have a better performance *in vivo.* Further experiments in simulated intestinal media should be performed in order to evaluate the effect of biliary salts on the size and colloidal stability of the formulations.

### 3.3 Preliminary conclusions on the evaluation of taxoid of formula Ib formulations

All the results concerning *the in vitro* behaviour in simulated GI fluids of the formulations for oral administration of taxoid of formula Ib, as well as the chemical stability in accelerated conditions, are summarized in the tables below.

**Table 5:**

| Summary of the *in vitro* behaviour of the formulations at 50 mg/g | | | | | |
|---|---|---|---|---|---|
| Formulation | Chemical Stability | Droplet size *in vitro* (2h at 37°C in gastric medium) | Homogeneity *in vitro* (2h at 37°C in gastric medium) | % released drug *in vitro* after 2h in gastric medium) | % released drug *in vitro* after 1h gastric + 2h Fassif |
| Labrasol | Not done | Not done | Good | 2% | 23% |
| SMES (5) | 7 months at 5°C | <30 nm | Good | >90% | 100% |

Since the *in vitro* behaviour of the 5 SMES is almost identical, the recommended SMES for further evaluation is the one containing Imwitor 988 as co-surfactant: indeed, this excipient is described as able to prevent the lipolysis inhibition that generally occurs with hydrophilic surfactants (such as Cremophor) and should allow the digestion of the lipid (Miglyol) for drug release and absorption. Since the absorption of taxoid of formula Ib is not a critical step, a delayed lipolysis (to enhance the uptake of intact droplets by the lymphatic pathway) is not desirable.

**Table 6:**

| Summary of the *in vitro* behaviour of the formulations at 100 mg/g | | | | | |
|---|---|---|---|---|---|
| Formulation | Chemical Stability | Droplet size *in vitro* (2h at 37°C in gastric medium) | Homogene ity *in vitro* (2h at 37°C *in* gastric medium) | % released drug *in vitro* after 2h in gastric medium) | % released drug *in vitro* after 1h gastric + 2h Fassif |
| Labrasol | >3 months at 40°C/75%R H | >1 µm | Good | 1-11% | 5-14% |
| SMES (5) | Not done | >1 µm (no microemulsion) | Not done | Not done | Not done |

At 100 mg/g, only Labrasol formulation exhibited a promising behaviour (in terms of release profile and droplet size).

### 4. Conclusions and further studies

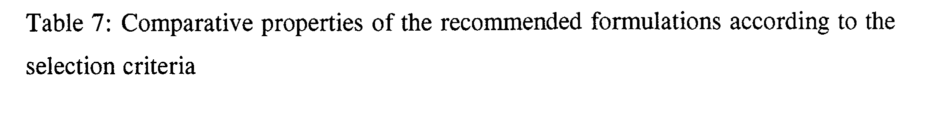

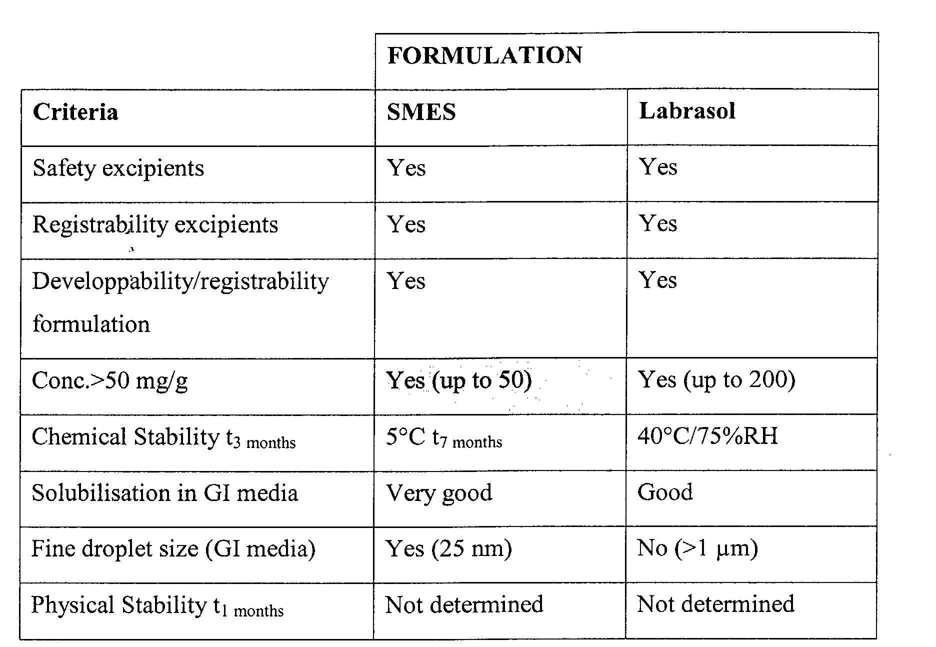

### Example 5: Taxoid of formula Ib - COMPARISON OF DIFFERENT FORMULATIONS IN BEAGLE DOGS

Three male Beagle dogs were tested with a dose of 0.5 mg/kg with the following formulations: Polysorbate 80; Self-microemulsifying system (SMES) at 50 mg/g (composition Cremophor EL 60%, Imwitor 988 20%, Miglyol 812N 20%); Nanocrystal suspension of 14C-taxoid of formula Ib. The Plasma radioactivity profiles were determined by LSC.

### Results:

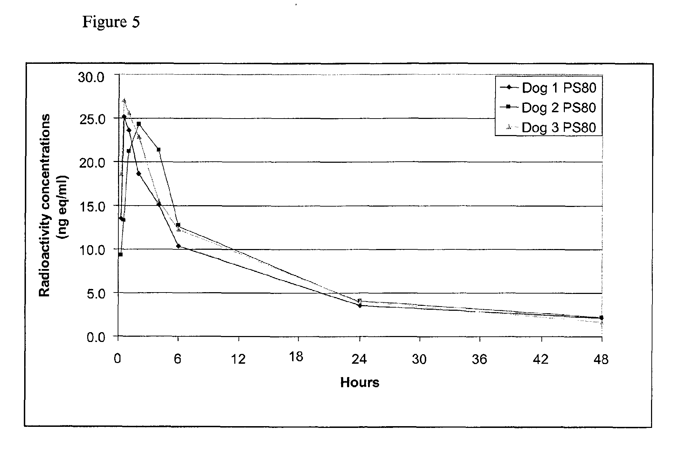

The plasma radioactivity concentration in the Beagle dogs after a single oral administration of C-14-taxoid of formula Ib at 0,5 mg/kg of a PS 80 formulation was determined (figure 5).

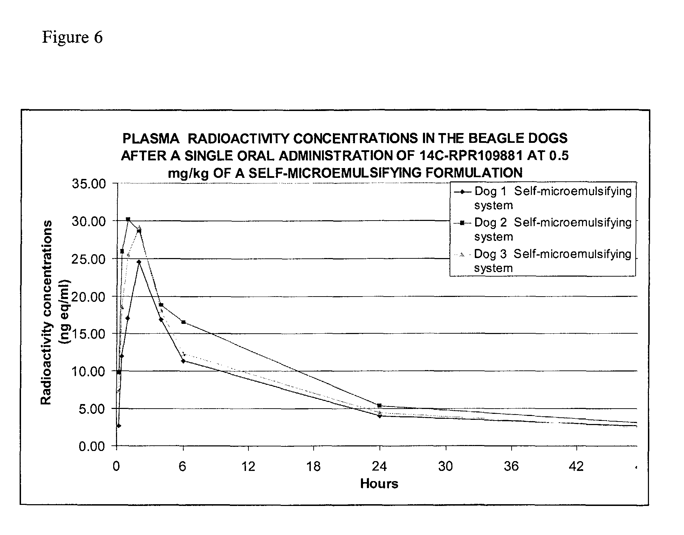

The plasma radioactivity concentration in the Beagle dogs after a single oral administration of C-14-taxoid of formula Ib at 0,5 mg/kg of the SMES formulation was determined (figure 6).

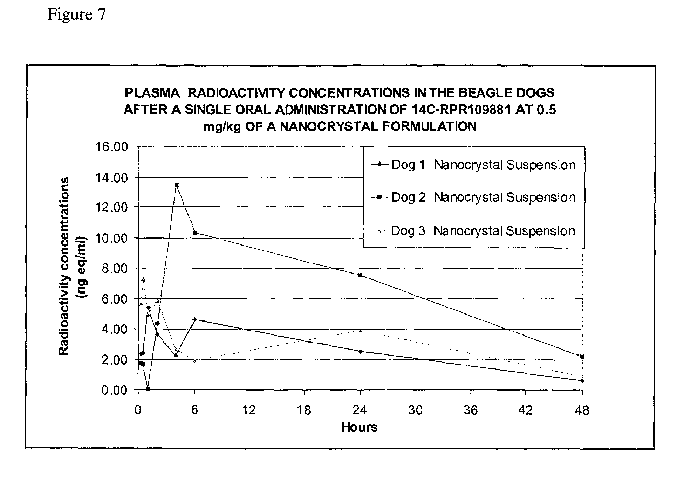

The plasma radioactivity concentration in the Beagle dogs after a single oral administration of C-14-taxoid of formula Ib at 0,5 mg/kg of a nanocrystal formulation was determined (figure 7).

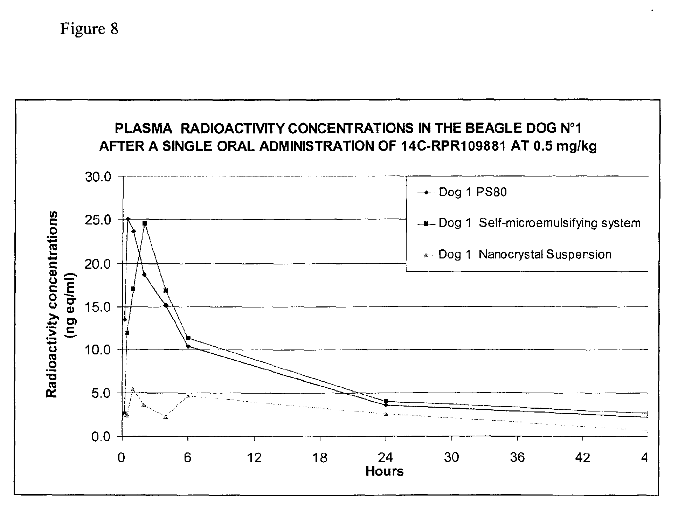

The plasma radioactivity concentration in the Beagle dog N°1 after a single oral administration of C-14-taxoid of formula Ib at 0.5 mg/kg was determined (figure 8).

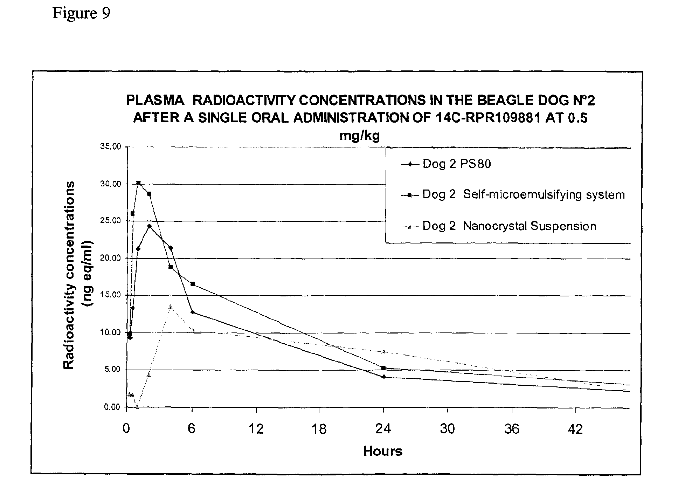

The plasma radioactivity concentration in the Beagle dog N°2 after a single oral administration of C-14-taxoid of formula Ib at 0.5 mg/kg was determined (figure 9).

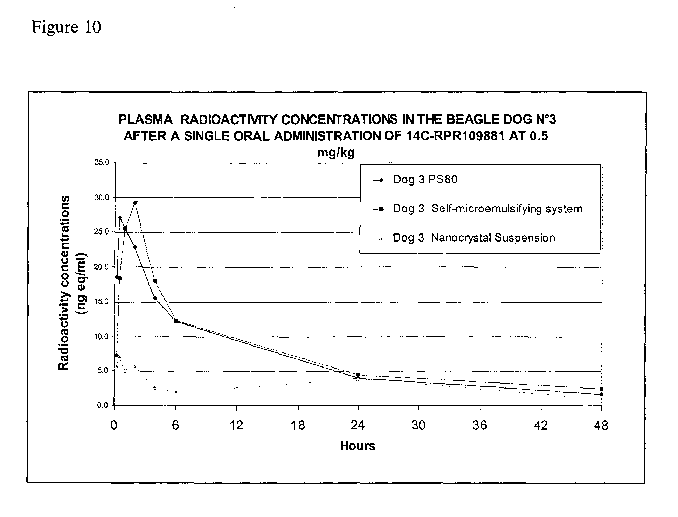

The plasma radioactivity concentration in the Beagle dog N°3 after a single oral administration ofC-14-taxoid of formula Ib at 0.5 mg/kg was determined (figure 10).

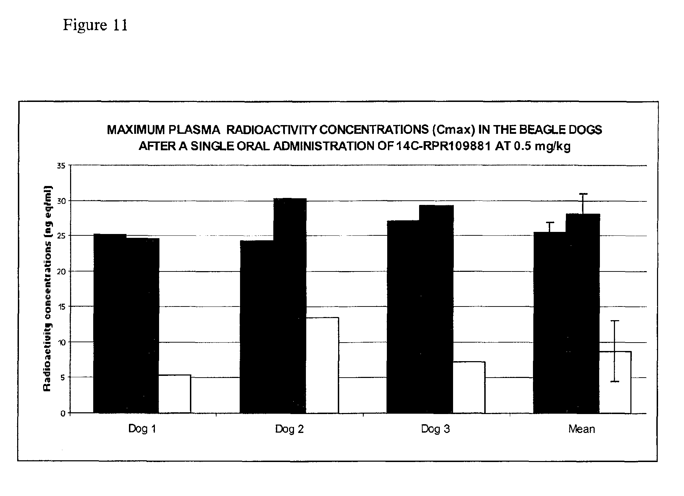

The maximum plasma radioactivity concentrations (Cmax) in the beagle dogs after a single oral administration of C-14-taxoid of formula Ib at 0.5 mg/kg were determined (figure 11). No difference was demonstrated in plasma radioactivity Cmax for PS80 and SMES formulations. Significant differences were demonstrated for PS80 or SMES and nanocrystal formulations.

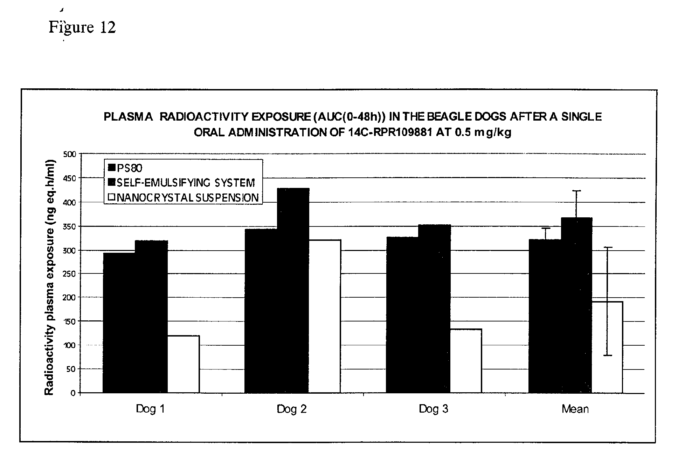

The² plasma radioactivity exposure (AUC (0-48 h)) in the Beagle dogs after a single oral administration of C-14-taxoid of formula Ib at 0.5 mg/kg was determined (figure 12). No difference was demonstrated in plasma radioactivity AUC for PS80 and SMES formulations. PS80 or SMES means AUC were 1.6-fold higher than that of nanocrystal.

The results can be summarized as follows:
- Rapid absorption of radioactivity (tmax 0.5-2h) and low variability of radioactivity concentrations (C.V <11% for Cmax) for PS80 and self-microemulsifying formulations were observed.
- Rapid to slow absorption (tmax 0.5-4h) of radioactivity and high variability of radioactivity concentrations (C.V. 49% for Cmax) for nanocrystal formulation were observed.
- No difference was demonstrated in plasma radioactivity Cmax and AUC for PS80 and self-microemulsifying formulations (320 ± 25 vs 366 ± 57 ng eq.h/mL, respectively)
- Plasma radioactivity mean Cmax and AUC for PS80 or self-microemulsifying formulations are at least 1.6-fold higher than that of nanocrystal.

## Claims

1. Self-emulsifying formulation for the oral administration of taxoids comprising at least one taxoid and at least one amphiphilic surfactant that is Labrasol®.

2. Self-emulsifying formulation as claimed in claim 1, wherein the formulation contains up to 200 mg taxoid per ml Labrasol®

3. Self-emulsifying formulation as claimed in claims 1 or 2, wherein the formulation contains between 50 and 100 mg taxoid per ml Labrasol®.

4. Self-emulsifying formulation for the oral administration of taxoids comprising at least one taxoid and at least one amphiphilic surfactant which is Labrasol® and at least one additional additive chosen from stabilizing agents, preservatives, agents which make it possible to adjust the viscosity, or agents that can modify the organoleptic properties.

5. Self-microemulsifying formulation for the oral administration of taxoids comprising at least one taxoid, at least one co-surfactant, at least one oil and a surfactant (Cremophor EL®).

6. Self-microemulsifying formulation for the oral administration of taxoids comprising at least one taxoid, at least one co-surfactant, at least one oil, one surfactant (Cremophor EL®) and at least one additional additive chosen from stabilizing agents, preservatives, agents which make it possible to adjust the viscosity, or agents that can modify the organoleptic properties.

7. Self-microemulsifying formulation as claimed in claims 5 or 6, wherein the co-surfactant is an amphiphilic surfactant with lipophilic character with an HLB of less than 10.

8. Self-microemulsifying formulation as claimed in claims 5 to 7, wherein the co-surfactant is chosen from Peceol®, Lauroglycol 129®, Capryol 90®, Maisine 35-1® and Imwitor 988®.

9. Self-microemulsifying formulation as claimed in claims 5 to 8, wherein the oil is Miglyol 812N®.

10. Self-microemulsifying formulation as claimed in claims 5 to 9, wherein the amount of co-surfactant is less than 50 % (weight percent).

11. Self-microemulsifying formulation as claimed in claims 5 to 10, wherein the oil concentration is less than 40 %.

12. Self-microemulsifying formulation as claimed in claims 5 to 11, wherein the ratio of surfactant to co-surfactant is 3:1 and the oil concentration is 20%.

13. Self-microemulsifying formulation as claimed in claims 5 to 12, wherein the taxoid concentration is not over 10% w/w.

14. Self-microemulsifying formulation as claimed in claim 13, wherein the taxoid concentration is from 1 to 50 mg/g.

15. Self-microemulsifying formulation as claimed in claims 5 to 14, wherein the formulation comprises 60% Cremophor EL, 20% Imwitor 888 and 20% Miglycol 812N (weight percent).

16. A process for preparing a formulation as claimed in claims 1 to 6, wherein there is prepared, where appropriate, the mixture of principal excipients, after heating, if necessary, in the case of the solid or semisolid excipients, and then, if necessary, the mixture with the additional additives, and then the taxoid and stirring is maintained in order to obtain a homogeneous mixture.
